(19) Europäisches Patentamt
European Patent Office
Office européen des brevets

(11) EP 0 589 839 B1

(12) **EUROPÄISCHE PATENTSCHRIFT**

(45) Veröffentlichungstag und Bekanntmachung des Hinweises auf die Patenterteilung:
**10.05.2000 Patentblatt 2000/19**

(51) Int. Cl.[7]: **C07D 307/83**, C07D 407/04, C08K 5/15, C08L 23/16, C08L 23/12

(21) Anmeldenummer: **93810652.3**

(22) Anmeldetag: **14.09.1993**

(54) **3-(Dihydrobenzofuran-5-yl)benzofuran-2-one als Stabilisatoren**

3-(Dihydrobenzofuran-5-yl)benzofuran-2-ones as stabilizers

3-(Dihydrobenzofuranne-5-yl)benzofuranne-2-ones stabilisateurs

(84) Benannte Vertragsstaaten:
**BE DE FR GB IT NL**

(30) Priorität: **23.09.1992 CH 298092**

(43) Veröffentlichungstag der Anmeldung:
**30.03.1994 Patentblatt 1994/13**

(73) Patentinhaber:
**Ciba Specialty Chemicals Holding Inc.**
**4057 Basel (CH)**

(72) Erfinder: **Nesvadba, Peter, Dr.**
**CH-1723 Marly (CH)**

(56) Entgegenhaltungen:

| | |
|---|---|
| **EP-A- 0 415 887** | **WO-A-80/01566** |
| **CH-A- 647 773** | **DE-A- 4 202 276** |
| **GB-A- 2 257 141** | **US-A- 3 862 133** |

- **PATENT ABSTRACTS OF JAPAN vol. 12, no. 316 (C-524)26. August 1988**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 589 839 B1

## Beschreibung

Die vorliegende Erfindung betrifft neue 3-(Dihydrobenzofuran-5-yl)benzofuran-2-one, Zusammensetzungen, enthaltend ein organisches Material, bevorzugt ein Polymer und die neuen Stabilisatoren, sowie die Verwendung derselben zum Stabilisieren von organischen Materialien gegen oxidativen, thermischen oder lichtinduzierten Abbau.

Einzelne 3-(Alkoxyphenyl)benzofuran-2-one wurden beispielsweise von M. Auger et al, Bull. Soc. Chim. Fr. 1970, 4024; L. Jurd, Aust. J. Chem., 31, 347 (1978) und C.S. Foote et al, J. Amer. Chem. Soc. 95, 586 (1973) und in DE-A-3 006 268 beschrieben.

Die Verwendung von einigen Benzofuran-2-onen als Stabilisatoren für organische Polymere ist beispielsweise aus US-A-4,325,863; US-A-4,338,244 und EP-A-415 887 bekannt.

Es wurde nun gefunden, dass eine ausgewahlte Gruppe solcher Benzofuran-2-one sich besonders gut als Stabilisatoren für organische Materialien, die gegen oxidativen, thermischen oder lichtinduzierten Abbau empfindlich sind, eignen.

Die vorliegende Erfindung betrifft daher Verbindungen der Formel (1)

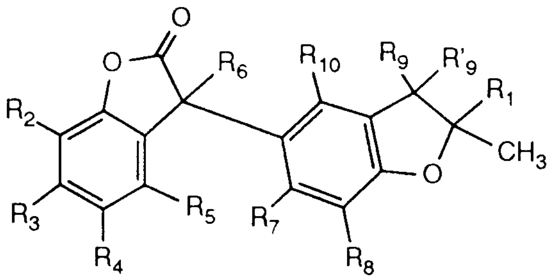

(1)

worin

$R_1$ Wasserstoff, $C_1$-$C_4$-Alkyl, unsubstituiertes oder durch $C_1$-$C_4$-Alkyl substituiertes Phenyl bedeutet,
$R_2$, $R_3$, $R_4$ und $R_5$ unabhängig voneinander Wasserstoff, Chlor, $C_1$-$C_{25}$-Alkyl, $C_7$-$C_9$-Phenylalkyl, unsubstituiertes oder durch $C_1$-$C_4$-Alkyl substituiertes Phenyl, unsubstituiertes oder durch $C_1$-$C_4$-Alkyl substituiertes $C_5$-$C_8$-Cycloalkyl; $C_1$-$C_{18}$-Alkoxy, $C_1$-$C_{18}$-Alkylthio, Hydroxy, $C_1$-$C_4$-Alkylamino, Di-($C_1$-$C_4$-alkyl)amino, $C_1$-$C_{25}$-Alkanoyloxy, $C_1$-$C_{25}$-Alkanoylamino, $C_3$-$C_{25}$-Alkenoyloxy, durch Sauerstoff, Schwefel oder $>N-R_{16}$ unterbrochenes $C_3$-$C_{25}$-Alkanoyloxy; $C_6$-$C_9$-Cycloalkylcarbonyloxy, Benzoyloxy oder durch $C_1$-$C_{12}$-Alkyl substituiertes Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Phenylring bilden, $R_4$ zusätzlich $-(CH_2)_n-COR_{11}$ darstellt, oder wenn $R_3$, $R_5$, $R_6$, $R_7$ und $R_{10}$ Wasserstoff sind, $R_4$ zusätzlich einen Rest der Formel (2)

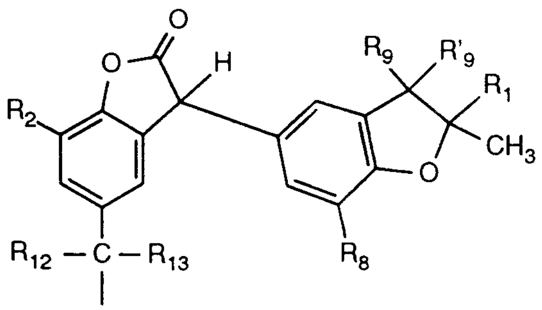

(2)

bedeutet,
$R_6$ Wasserstoff oder einen Rest der Formel (3)

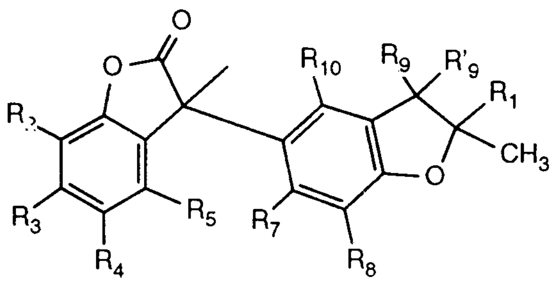

(3)

darstellt, wobei $R_4$ nicht einen Rest der Formel (2) bedeutet,

$R_7$ und $R_8$ unabhängig voneinander Wasserstoff oder $C_1$-$C_4$-Alkyl darstellen,

$R_9$ und $R'_9$ Wasserstoff, $C_1$-$C_4$-Alkyl oder Phenyl bedeuten, mit der Bedingung, dass mindestens einer der Reste $R_9$ und $R'_9$ Wasserstoff ist,

$R_{10}$ Wasserstoff oder $C_1$-$C_4$-Alkyl darstellt,

$R_{11}$ Hydroxy, $[-O^{\ominus}\frac{1}{r}M^{r+}]$, $C_1$-$C_{18}$-Alkoxy oder

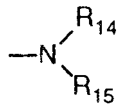

bedeutet,

$R_{12}$ und $R_{13}$ unabhängig voneinander Wasserstoff, $CF_3$, $C_1$-$C_{12}$-Alkyl oder Phenyl darstellen, oder $R_{12}$ und $R_{13}$ zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden;

$R_{14}$ und $R_{15}$ unabhängig voneinander Wasserstoff oder $C_1$-$C_{18}$-Alkyl darstellen,

$R_{16}$ Wasserstoff oder $C_1$-$C_8$-Alkyl bedeutet,

M ein r-wertiges Metallkation ist,

n 0, 1 oder 2 und

r 1,2 oder 3 bedeutet.

Alkyl mit bis zu 25 Kohlenstoffatomen bedeutet einen verzweigten oder unverzweigten Rest wie beispielsweise Methyl, Ethyl, Propyl, Isopropyl, n-Butyl, sec-Butyl, Isobutyl, tert-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl oder Docosyl. Eine der bevorzugten Bedeutungen von $R_2$ und $R_4$ ist beispielweise $C_1$-$C_{18}$-Alkyl. Eine besonders bevorzugte Bedeutung von $R_4$ ist $C_1$-$C_4$-Alkyl.

$C_7$-$C_9$-Phenylalkyl bedeutet beispielsweise Benzyl, α-Methylbenzyl, α,α-Dimethylbenzyl oder 2-Phenylethyl. Benzyl ist bevorzugt.

Durch $C_1$-$C_4$-Alkyl substituiertes Phenyl, das vorzugsweise 1 bis 3, insbesondere 1 oder 2 Alkylgruppen enthält, bedeutet beispielsweise o-, m- oder p-Methylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-6-ethylphenyl, 4-tert-butylphenyl, 2-Ethylphenyl oder 2,6-Diethylphenyl.

Unsubstituiertes oder durch $C_1$-$C_4$-Alkyl substituiertes $C_5$-$C_8$-Cycloalkyl bedeutet beispielsweise Cyclopentyl, Methylcyclopentyl, Dimethylcyclopentyl, Cyclohexyl, Methylcyclohexyl, Dimethylcyclohexyl, Trimethylcyclohexyl, tert-Butylcyclohexyl, Cycloheptyl oder Cyclooctyl. Bevorzugt ist Cyclohexyl und tert-Butylcyclohexyl.

Alkoxy mit bis zu 18 Kohlenstoffatomen bedeutet einen verzweigten oder unverzweigten Rest wie beispielsweise Methoxy, Ethoxy, Propoxy, Isopropoxy, n-Butoxy, Isobutoxy, Pentoxy, Isopentoxy, Hexoxy, Heptoxy, Octoxy, Decyloxy, Tetradecyloxy, Hexadecyloxy oder Octadecyloxy.

Alkylthio mit bis zu 18 Kohlenstoffatomen bedeutet einen verzweigten oder unverzweigten Rest wie beispielsweise Methylthio, Ethylthio, Propylthio, Isopropylthio, n-Butylthio, Isobutylthio, Pentylthio, Isopentylthio, Hexylthio, Heptylthio, Octylthio, Decylthio, Tetradecylthio, Hexadecylthio oder Octadecylthio.

Alkylamino mit bis zu 4 Kohlenstoffatomen bedeutet einen verzweigten oder unverzweigten Rest wie beispielsweise Methylamino, Ethylamino, Propylamino, Isopropylamino, n-Butylamino, Isobutylamino oder tert-Butylamino.

Di-($C_1$-$C_4$)alkylamino bedeutet auch, dass die beiden Reste unabhängig voneinander verzweigt oder unverzweigt sind wie beispielsweise Dimethylamino, Methylethylamino, Diethylamino, Methyl-n-propylamino, Methylisopropylamino, Methyl-n-butylamino, Methylisobutylamino, Ethylisopropylamino, Ethyl-n-butylamino, Ethylisobutylamino, Ethyl-tert-butylamino, Diethylamino, Diisopropylamino, Isopropyl-n-butylamino, Isopropylisobutylamino, Di-n-butylamino oder Di-isobutylamino.

Alkanoyloxy mit bis zu 25 Kohlenstoffatomen bedeutet einen verzweigten oder unverzweigten Rest wie beispielsweise Formyloxy, Acetyloxy, Propionyloxy, Butanoyloxy, Pentanoyloxy, Hexanoyloxy, Heptanoyloxy, Octanoyloxy, Nonanoyloxy, Decanoyloxy, Undecanoyloxy, Dodecanoyloxy, Tridecanoyloxy, Tetradecanoyloxy, Pentadecanoyloxy, Hexadecanoyloxy, Heptadecanoyloxy, Octadecanoyloxy, Eicosanoyloxy oder Docosanoyloxy.

Alkanoylamino mit bis zu 25 Kohlenstoffatomen bedeutet einen verzweigten oder unverzweigten Rest wie beispielsweise Formylamino, Acetylamino, Propionylamino, Butanoylamino, Pentanoylamino, Hexanoylamino, Heptanoylamino, Octanoylamino, Nonanoylamino, Decanoylamino, Undecanoylamino, Dodecanoylamino, Tridecanoylamino, Tetradecanoylamino, Pentadecanoylamino, Hexadecanoylamino, Heptadecanoylamino, Octadecanoylamino, Eicosanoylamino oder Docosanoylamino.

Alkenoyloxy mit 3 bis 25 Kohlenstoffatomen bedeutet einen verzweigten oder unverzweigten Rest wie beispielsweise Propenoyloxy, 2-Butenoyloxy, 3-Butenoyloxy, Isobutenoyloxy, n-2,4-Pentadienoyloxy, 3-Methyl-2-butenoyloxy, n-2-Octenoyloxy, n-2-Dodecenoyloxy, iso-Dodecenoyloxy, Oleoyloxy, n-2-Octadecenoyloxy oder n-4-Octadecenoyloxy.

Durch Sauerstoff, Schwefel oder $>N-R_{16}$ unterbrochenes $C_3$-$C_{25}$-Alkanoyloxy bedeutet beispielsweise $CH_3-O-CH_2COO-$, $CH_3-S-CH_2COO-$, $CH_3-NH-CH_2COO-$, $CH_3-N(CH_3)-CH_2COO-$, $CH_3-O-CH_2CH_2-O-CH_2COO-$, $CH_3-(O-CH_2CH_2-)_2O-CH_2COO-$, $CH_3-(O-CH_2CH_2-)_3O-CH_2COO-$ oder $CH_3-(O-CH_2CH_2-)_4O-CH_2COO-$.

$C_6$-$C_9$-Cycloalkylcarbonyloxy bedeutet beispielsweise Cyclopentylcarbonyloxy, Cyclohexylcarbonyloxy, Cycloheptylcarbonyloxy oder Cyclooctylcarbonyloxy. Cyclohexylcarbonyloxy ist bevorzugt.

Durch $C_1$-$C_{12}$-Alkyl substituiertes Benzoyloxy bedeutet beispielsweise o-, m- oder p-Methylbenzoyloxy, 2,3-Dimethylbenzoyloxy, 2,4-Dimethylbenzoyloxy, 2,5-Dimethylbenzoyloxy, 2,6-Dimethylbenzoyloxy, 3,4-Dimethylbenzoyloxy, 3,5-Dimethylbenzoyloxy, 2-Methyl-6-ethylbenzoyloxy, 4-tert-Butylbenzoyloxy, 2-Ethylbenzoyloxy, 2,4,6-Trimethylbenzoyloxy, 2,6-Dimethyl-4-tert-butylbenzoyloxy oder 3,5-Di-tert-butylbenzoyloxy.

Durch $C_1$-$C_4$-Alkyl substituierter $C_5$-$C_8$-Cycloalkylidenring, der vorzugsweise 1 bis 3, insbesondere 1 oder 2 verzweigte oder unverzweigte Alkylgruppen-Reste enthält, bedeutet beispielsweise Cyclopentyliden, Methylcyclopentyliden, Dimethylcyclopentyliden, Cyclohexyliden, Methylcyclohexyliden, Dimethylcyclohexyliden, Trimethylcyclohexyliden, tert-Butylcyclohexyliden, Cycloheptyliden oder Cyclooctyliden. Bevorzugt ist Cyclohexyliden und tert-Butylcyclohexyliden.

Ein ein-, zwei- oder drei-wertiges Metallkation ist vorzugsweise ein Alkalimetall-, Erdalkalimetall- oder Aluminium-Kation, beispielsweise $Na^+$, $K^+$, $Mg^{++}$, $Ca^{++}$ oder $Al^{+++}$.

Bevorzugt sind Verbindungen der Formel (1), worin

$R_1$ Wasserstoff, $C_1$-$C_4$-Alkyl oder Phenyl bedeutet,
$R_2$, $R_3$, $R_4$ und $R_5$ unabhängig voneinander Wasserstoff, Chlor, $C_1$-$C_{18}$-Alkyl, Benzyl, Phenyl, $C_5$-$C_8$-Cycloalkyl, $C_1$-$C_{18}$-Alkoxy, $C_1$-$C_{18}$-Alkylthio, $C_1$-$C_{18}$-Alkanoyloxy, $C_1$-$C_{18}$-Alkanoylamino, $C_3$-$C_{18}$-Alkenoyloxy oder Benzoyloxy darstellen, oder ferner die Reste $R_2$ und $R_3$ oder die Reste $R_4$ und $R_5$ zusammen mit den Kohlenstoffatomen, an die sie gebunden sind, einen Phenylring bilden, oder wenn $R_3$, $R_5$, $R_6$, $R_7$ und $R_{10}$ Wasserstoff sind, $R_4$ zusätzlich einen Rest der Formel (2) bedeutet,
$R_9$ und $R'_9$ Wasserstoff oder $C_1$-$C_4$-Alkyl darstellen, mit der Bedingung, dass mindestens einer der Reste $R_9$ und $R'_9$ Wasserstoff ist, und
$R_{12}$ und $R_{13}$ entweder Methylgruppen sind, oder zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden.

Bevorzugt sind auch Verbindungen der Formel (1), worin mindestens zwei der Reste $R_2$, $R_3$, $R_4$ und $R_5$ Wasserstoff sind.

Ebenfalls bevorzugt sind Verbindungen der Formel (1), worin $R_3$ und $R_5$ Wasserstoff sind.

Besonders bevorzugt sind Verbindungen der Formel (1), worin

$R_1$ Wasserstoff oder $C_1$-$C_4$-Alkyl bedeutet,
$R_2$ und $R_4$ unabhängig voneinander Wasserstoff, $C_1$-$C_{18}$-Alkyl, Benzyl, Phenyl, $C_5$-$C_8$-Cycloalkyl oder $C_1$-$C_{18}$-Alkoxy darstellen, oder wenn $R_3$, $R_5$, $R_6$, $R_7$ und $R_{10}$ Wasserstoff sind, $R_4$ zusätzlich einen Rest der Formel (2) bedeutet,

$R_3$ und $R_5$ unabhängig voneinander Wasserstoff oder $C_1$-$C_4$-Alkyl darstellen,
$R_6$ Wasserstoff oder einen Rest der Formel (3) darstellt, wobei $R_4$ nicht einen Rest der Formel (2) bedeutet,
$R_7$, $R_8$, $R_9$, $R'_9$ und $R_{10}$ Wasserstoff sind, und
$R_{12}$ und $R_{13}$ entweder Methylgruppen sind, oder zusammen mit dem C-Atom, an das sie gebunden sind, einen $C_5$-$C_8$-Cycloalkylidenring bilden.

Speziell von besonderem Interesse sind Verbindungen der Formel (1), worin

$R_1$ Wasserstoff oder Methyl ist,
$R_2$ Wasserstoff oder $C_1$-$C_{18}$-Alkyl bedeutet,
$R_3$, $R_5$, $R_7$, $R_8$, $R_9$, $R'_9$ und $R_{10}$ Wasserstoff sind,
$R_4$ Wasserstoff oder $C_1$-$C_4$-Alkyl darstellt, oder wenn $R_6$ Wasserstoff ist, $R_4$ zusätzlich einen Rest der Formel (2) bedeutet,
$R_6$ Wasserstoff oder einen Rest der Formel (3) darstellt, wobei $R_4$ nicht einen Rest der Formel (2) bedeutet, und
$R_{12}$ und $R_{13}$ entweder Methylgruppen sind, oder zusammen mit dem C-Atom, an das sie gebunden sind, einen Cyclohexylidenring bilden.

Die erfindungsgemässen Verbindungen der Formel (1) können auf an sich bekannte Weise hergestellt werden.

Beispielsweise, und dies ist bevorzugt, wird ein Phenol der Formel (4),

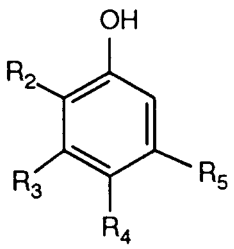

(4)

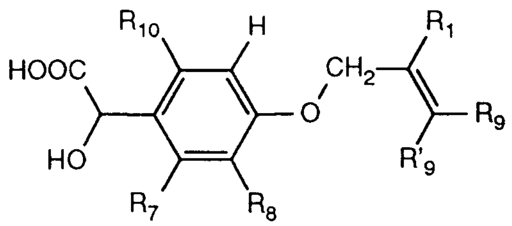

(5)

worin $R_2$, $R_3$, $R_4$ und $R_5$ die angegebenen Bedeutungen haben, mit einem am Phenylring substituierten Allyloxy-Mandelsäure-Derivat der Formel (5), worin $R_1$, $R_7$, $R_8$, $R_9$, $R'_9$ und $R_{10}$ die angegebenen Bedeutungen haben, bei erhöhter Temperatur, insbesondere Temperaturen von 130 bis 200°C in der Schmelze oder in einem Lösungsmittel gegebenenfalls unter leichtem Vakuum, zu Verbindungen der Formel (6)

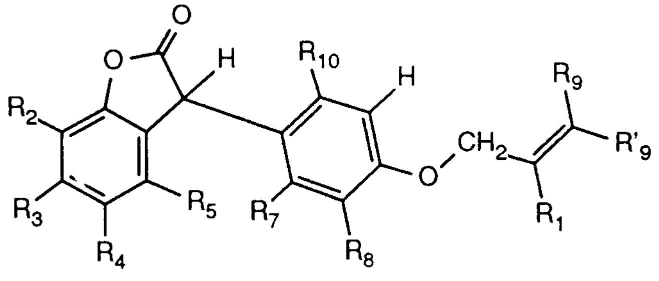

(6)

umgesetzt. Bevorzugt wird die Reaktion in einem Lösungsmittel wie beispielsweise Essigsäure oder Ameisensäure in einem Temperaturbereich von 50 bis 130°C durchgeführt. Die Reaktion kann durch Zusatz einer Säure wie Salzsäure, Schwefelsäure oder Methansulfonsäure katalysiert werden. Die Umsetzung kann z.B. in der Weise durchgeführt werden, wie sie in den in der Beschreibungseinleitung angegebenen Literaturstellen beschrieben ist.

Die am Phenylring substituierten Mandelsäuren der Formel (5) sind in der Literatur bekannt oder können beispielsweise gemäss W. Bradley et al, J. Chem. Soc. 1956, 1622; EP-A-146269, EP-B-182507 (Beispiel 1, Seite 4) oder DE-A-2 944 295 in analoger Weise hergestellt werden.

Die Phenole der Formel (4) sind ebenfalls bekannt oder können nach an sich bekannten Verfahren erhalten werden.

Bisphenolverbindungen der Formel (7)

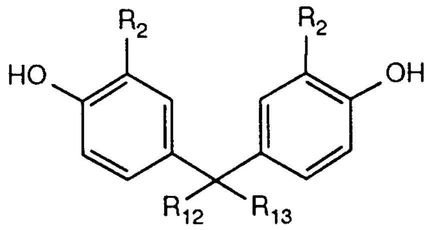

(7)

können gemäss Houben-Weyl, Methoden der organischen Chemie, Band 6/1c, 1030, hergestellt werden.

Die Verbindungen der Formel (6) werden unter Claisen-Umlagerungsbedingungen, bei erhöhter Temperatur, insbesondere Temperaturen von 200 bis 240°C in der Schmelze oder in einem Lösungsmittel zu den Verbindungen der Formel (8)

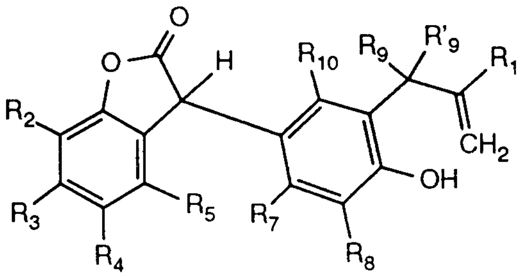

(8)

umgelagert. Diese Verbindungen können isoliert oder direkt ohne Reinigung in einem Lösungsmittel, wie beispielsweise Essigsäure, mit einer katalytischen Menge einer starken Säure, wie beispielsweise Methansulfonsäure, bei erhöhter Temperatur, insbesondere Temperaturen von 50 bis 130°C zu den erfindungsgemässen Verbindungen der Formel (1) cyclisiert werden.

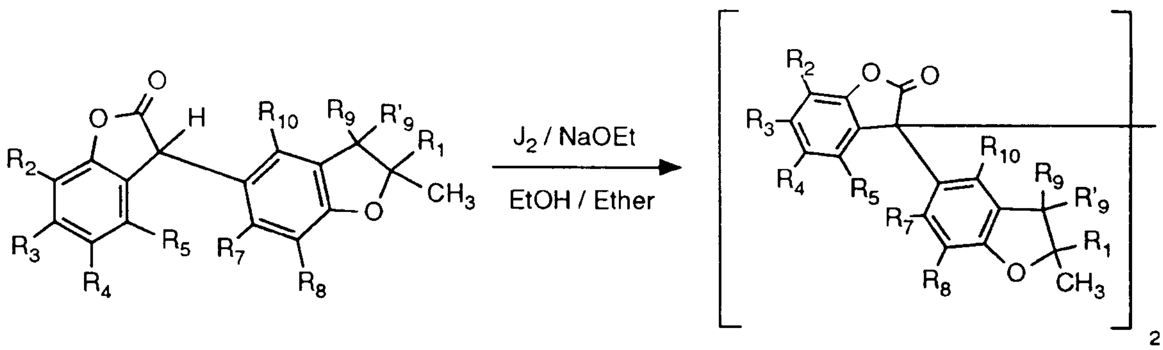


(9) (10)

Die Dimerisierung der Verbindungen der Formel (9) zur Herstellung von Verbindungen der Formel (1), worin $R_6$ eine Gruppe der Formel (3) ist [Verbindungen der Formel (10)] erfolgt durch Oxidation mit beispielsweise Jod unter basischen Bedingungen in einem organischen Lösungsmittel bei Raumtemperatur. Als Base eignet sich besonders Natriumethylat, als Lösungsmittel Ethanol und Diethylether.

Die erfindungsgemässen Verbindungen der Formel (1) eignen sich zum Stabilisieren von organischen Materialien gegen thermischen, oxidativen oder lichtinduzierten Abbau.

Beispiele für derartige Materialien sind:

1. Polymere von Mono- und Diolefinen, beispielsweise Polypropylen, Polyisobutylen, Polybuten-1, Poly-4-methylpenten- 1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).

Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:

a) radikalisch (gewöhnlich bei hohem Druck und hoher Temperatur).

b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder $\pi$- oder $\sigma$-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.

4. Kohlenwasserstoffharze (z.B. $C_5$-$C_9$) inklusive hydrierte Modifikationen davon (z.B. Klebrigmacherharze) und Mischungen von Polyalkylenen und Stärke.

5. Polystyrol, Poly-(p-methylstyrol), Poly-($\alpha$-methylstyrol).

6. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzahigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

7. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.

8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

9. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.

10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

11. Polymere, die sich von ungesättigten Alkoholen und Aminen bzw. deren Acylderivaten oder Acetalen ableiten, wie Polyvinylalkohol, Polyvinylacetat, -stearat, - benzoat, -maleat, Polyvinylbutyral, Polyallylphthalat, Polyallylmelamin; sowie deren Copolymere mit in Punkt 1 genannten Olefinen.

12. Homo- und Copolymere von cyclischen Ethern, wie Polyalkylenglykole, Polyethylenoxyd, Polypropylenoxyd oder deren Copolymere mit Bisglycidylethern.

13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

14. Polyphenylenoxide und -sulfide und deren Mischungen mit Styrolpolymeren oder Polyamiden.

15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

17. Polyharnstoffe, Polyimide, Polyamid-imide und Polybenzimidazole.

18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

19. Polycarbonate und Polyestercarbonate.

20. Polysulfone, Polyethersulfone und Polyetherketone.

21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

22. Trocknende und nicht-trocknende Alkydharze.

23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

24. Vernetzbare Acrylharze, die sich von substituierten Acrylsäureestern ableiten, wie z.B. von Epoxyacrylaten, Urethan-acrylaten oder Polyester-acrylaten.

25. Alkydharze, Polyesterharze und Acrylatharze, die mit Melaminharzen, Harnstoffharzen, Polyisocyanaten oder Epoxidharzen vernetzt sind.

26. Vernetzte Epoxidharze, die sich von Polyepoxiden ableiten, z.B. von Bis-glycidylethern oder von cycloaliphatischen Diepoxiden.

27. Natürliche Polymere, wie Cellulose, Naturkautschuk, Gelatine, sowie deren polymerhomolog chemisch abgewandelte Derivate, wie Celluloseacetate, -propionate und - butyrate, bzw. die Celluloseether, wie Methylcellulose; sowie Kolophoniumharze und Derivate.

28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

29. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

30.Wässrige Emulsionen natürlicher oder synthetischer Kautschuke, wie z.B. Naturkautschuk-Latex oder Latices von carboxylierten Styrol-Butadien-Copolymeren.

Weitere Gegenstände der Erfindung sind daher auch Zusammensetzungen enthaltend ein dem oxidativen, thermischen oder lichtinduzierten Abbau unterworfenes organisches Material und mindestens eine Verbindung der Formel (1).

Bevorzugte organische Materialien sind Polymere, z.B. synthetische Polymere, insbesondere thermoplastische Polymere. Besonders bevorzugt sind Polyacetale oder Polyolefine, z.B. Polypropylen oder Polyethylen.

Besonders hervorzuheben ist die Wirkung der erfindungsgemässen Verbindungen gegen thermischen und oxidativen Abbau, vor allem bei thermischer Belastung, wie sie bei der Verarbeitung von Thermoplasten auftritt. Die erfindungsgemässen Verbindungen sind daher hervorragend als Verarbeitungsstabilisatoren einzusetzen.

Vorzugsweise werden die Verbindungen der Formel (1) dem zu stabilisierenden Material in Mengen von 0,0005 bis 5 %, insbesondere 0,001 bis 2 %, beispielsweise 0,01 bis 2 %, zugesetzt, bezogen auf das Gewicht des zu stabilisierenden organischen Materials.

Zusätzlich zu den Verbindungen der Formel (1) können die erfindungsgemässen Zusammensetzungen weitere Costabilisatoren enthalten, wie beispielsweise die folgenden:

<u>1. Antioxidantien</u>

<u>1.1. Alkylierte Monophenole</u>, z.B. 2,6-Di-tert-butyl-4-methylphenol, 2-butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-($\alpha$-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.

1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.

1.3. Hydrochinone und alkylierte Hydrochinone, z.B. 2,6-Di-tert-butyl-4-methoxyphenol, 2,5-Di-tert-butylhydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tertbutyl-4-hydroxyanisol, 3,5-Di-tert-butyl-4-hydroxyphenyl-stearat, Bis-(3,5-di-tert-butyl4-hydroxyphenyl)adipat.

1.4. Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3,6-di-sec.-amylphenol), 4,4'-Bis-(2,6-di-methyl-4-hydroxyphenyl)-disulfid.

1.5. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methyl-cyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonylphenol], 2,2'-Methylen-bis-[6-(α,α-di-methylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.

1.6. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-di-thioterephthalat, Bis-(3,5-di-tert-butyl-4-hydroxybenzyl)-sulfid, Isooctyl-3,5-di-tert-butyl-4-hydroxybenzyl-mercaptoacetat.

1.7. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.

1.8. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.

1.9. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.

1.10. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.

1.11. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.

1.12. Ester der β-(3,5-Di-tert-butyl-4-hydroxyphenyl)-propionsäure mit ein- oder mehr wertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.13. Ester der β-(5-tert-Butyl-4-hydroxy-3-methylphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.14. Ester der β-(3,5-Dicyclohexyl-4-hydroxyphenyl)-propionsäure mit ein- oder mehr wertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der 3,5-Di-tert-butyl-4-hydroxyphenylessigsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.16. Amide der β-(3,5-Di-tert-butyl-4-hydroxyphenyl)-propionsäure, wie z.B. N,N'-Bis-(3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.

2. UV-Absorber und Lichtschutzmittel

2.1. 2-(2'-Hydroxyphenyl)-benztriazole, wie z.B. 2-(2'-Hydroxy-5'-methylphenyl)-benztriazol, 2-(3',5'-Di-tert-butyl-2'-hydroxyphenyl)-benztriazol, 2-(5'-tert-Butyl-2'-hydroxyphenyl)-benztriazol, 2-(2'-Hydroxy-5'-(1,1,3,3-tetramethylbutyl)phenyl)-benztriazol, 2-(3',5'-Di-tert-butyl-2'-hydroxyphenyl)-5-chlor-benztriazol, 2-(3'-tert-Butyl-2'-hydroxy-5'-methylphenyl)-5-chlor-benztriazol, 2-(3'-sec-Butyl-5'-tert-butyl-2'-hydroxyphenyl)-benztriazol, 2-(2'-Hydroxy-4'-octoxyphenyl)-benztriazol, 2-(3',5'-Di-tert-amyl-2'-hydroxyphenyl)-benztriazol, 2-(3',5'-Bis-(α,α-dimethylbenzyl)-2'-hydroxyphenyl)-benztriazol, Mischung aus 2-(3'-tert-Butyl-2'-hydroxy-5'-(2-octyloxycarbonylethyl)phenyl)-5-chlor-benztriazol, 2-(3'-tert-Butyl-5'-[2-(2-ethylhexyloxy)-carbonylethyl]-2'-hydroxyphenyl)-5-chlor-benztriazol, 2-(3'-tert-Butyl-2'-hydroxy-5'-(2-methoxycarbonylethyl)phenyl)-5-chlor-benztriazol, 2-(3'-tert-Butyl-2'-hydroxy-5'-(2-methoxycarbonylethyl)phenyl)-benztriazol, 2-(3'-tert-Butyl-2'-hydroxy-5'-(2-octyloxycarbonylethyl)phenyl)-benztriazol, 2-(3'-tert-Butyl-5'-[2-(2-ethylhexyloxy)carbonylethyl]-2'-hydroxyphenyl)-benztriazol, 2-(3'-Dodecyl-2'-hydroxy-5'-methylphenyl)-benztriazol, und 2-(3'-tert-Butyl-2'-hydroxy-5'-(2-isooctyloxycarbonylethyl)phenyl-benztriazol, 2,2'-Methylen-bis[4-(1,1,3,3-tetramethylbutyl)-6-benztriazol-2-yl-phenol]; Umesterungsprodukt von 2-[3'-tert-Butyl-5'-(2-methoxycarbonylethyl)-2'-hydroxy-phenyl]-benztriazol mit Polyethylenglycol 300; $[R-CH_2CH_2-COO(CH_2)_3]_2$ mit R = 3'-tert-Butyl-4'-hydroxy-5'-2H-benztriazol-2-yl-phenyl.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.

2.4. Acrylate, wie z.B. α-Cyan-β,β-diphenylacrylsäure-ethylester bzw. - isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin

oder N-Cyclohexyl-diethanolamin, Nickeldibutyldithiocarbamat, Nickelsalze von 4-Hydroxy-3,5-di-tert-butylbenzylphosphonsäure-monoalkylestern, wie von Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1,2,2,6,6-pentamethylpipendyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)- 1,3 ,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.

2.7. Oxalsäurediamide, wie z.B. 4,4'-Di-octyloxy-oxanilid, 2,2'-Di-octyloxy-5,5'-di-tert-butyl-oxanilid, 2,2'-Di-dodecyloxy-5,5,di-tert-butyl-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o-und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert-butyl-4-hydroxyphenylpropionyl)-hydrazin, 3-Salicyloylamino-1,2,4-triazol, Bis-(benzyliden)-oxalsäuredihydrazid, Oxanilid, Isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythrit-diphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis-(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-di-benz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.

8. Nukleierungsmittel, wie z.B. 4-tert-Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.

10. Sonstige Zusätze, wie z.B. Weichmacher, Gleitmittel, Emulgatoren, Pigmente, Optische Aufheller, Flammschutzmittel, Antistatika, Treibmittel.

Die Costabilisatoren werden beispielsweise in Konzentrationen von 0,01 bis 10 %, bezogen auf das Gesamtgewicht des zu stabilisierenden Materials, zugesetzt.

Die erfindungsgemässen Verbindungen der Formel (1) können insbesondere zusammen mit phenolischen Antioxidantien eingesetzt werden. Die erfindungsgemässen Zusammensetzungen enthalten daher vorzugsweise neben Verbindungen der Formel (1) phenolische Antioxidantien, insbesondere solche, wie sie in den obigen Punkten 1.1 bis 1.16 aufgelistet sind.

Andere bevorzugte Zusammensetzungen enthalten neben den Verbindungen der Formel (1) mindestens ein organisches Phosphit oder Phosphonit.

Die Einarbeitung der Verbindungen der Formel (1) sowie gegebenenfalls weiterer Additive in das polymere, organische Material erfolgt nach bekannten Methoden, beispielsweise vor oder während der Formgebung oder auch durch Aufbringen der gelösten oder dispergierten Verbindungen auf das polymere, organische Material, gegebenenfalls unter nachträglichem Verdunsten des Lösungsmittels. Die Verbindungen der Formel (1) können auch in Form eines Masterbatches, der diese beispielsweise in einer Konzentration von 2,5 bis 25 Gew.-% enthält, den zu stabilisierenden Materialien zugesetzt werden.

Die Verbindungen der Formel (1) können auch vor oder während der Polymerisation oder vor der Vernetzung zugegeben werden.

Die Verbindungen der Formel (1) können in reiner Form oder in Wachsen, Oelen oder Polymeren verkapselt in das zu stabilisierende Material eingearbeitet werden.

Die Verbindungen der Formel (1) können auch auf das zu stabilisierende Polymer aufgesprüht werden. Sie sind in der Lage, andere Zusätze (z.B. die oben angegebenen herkömmlichen Additive) bzw. deren Schmelzen zu verdünnen, so dass sie auch zusammen mit diesen Zusätzen auf das zu stabilisierende Polymer aufgesprüht werden können. Besonders vorteilhaft ist die Zugabe durch Aufsprühen während der Desaktivierung der Polymerisationskatalysatoren, wobei z.B. der zur Desaktivierung verwendete Dampf zum Versprühen verwendet werden kann.

Bei kugelförmig polymerisierten Polyolefinen kann es z.B. vorteilhaft sein, die Verbindungen der Formel (1), gegebenenfalls zusammen mit anderen Additiven, durch Aufsprühen zu applizieren.

Eine bevorzugte Ausführungsform der vorliegenden Erfindung ist daher die Verwendung von Verbindungen der Formel (1) zum Stabilisieren von organischen Materialien gegen oxidativen, thermischen oder lichtinduzierten Abbau.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Die vorliegende Erfindung betrifft auch ein Verfahren zum Stabilisieren eines organischen Materials gegen oxidativen, thermischen oder lichtinduzierten Abbau, das dadurch gekennzeichnet ist, dass man diesem mindestens eine Verbindung der Formel (1) einverleibt oder auf dieses aufbringt.

Wie bereits hervorgehoben, werden die erfindungsgemässen Verbindungen besonders vorteilhaft als Stabilisatoren in Polyolefinen eingesetzt, vor allem als Thermostabilisatoren. Ausgezeichnete Stabilisierung wird z.B. dann erhalten, wenn man sie in Kombination mit organischen Phosphiten oder Phosphoniten einsetzt. Dabei weisen die erfindungsgemässen Verbindungen den Vorteil auf, dass sie bereits in ausserordentlich geringen Mengen wirksam sind. Sie werden z.B. in Mengen von 0,0001 bis 0,015, insbesondere 0,0001 bis 0,008 Gew. % bezogen auf das Polyolefin, eingesetzt. Das organische Phosphit oder Phosphonit wird zweckmässig in einer Menge von 0,01 bis 2, insbesondere 0,01 bis 1 Gew. %, ebenfalls bezogen auf das Polyolefin, eingesetzt. Als organische Phosphite bzw. Phosphonite werden vorzugsweise solche eingesetzt, wie sie in DE-A-4 202 276 beschrieben sind. Siehe dort insbesondere die Patentansprüche, die Beispiele sowie die Seiten 5, letzter Absatz bis Seite 8. Besonders zweckmässige Phosphite und Phosphonite sind auch Punkt 4 der obigen Auflistung von Costabilisatoren zu entnehmen.

Die folgenden Beispiele erläutern die Erfindung weiter. Angaben in Teilen oder Prozenten beziehen sich auf

das Gewicht.

Beispiel 1: Herstellung von 5,7-Di-tert-butyl-3-(2-methyl-dihydrobenzofuran-5-yl)benzofuran-2-on (Verbindung (101), Tabelle 1).

3,80 g (10,0 mMol) 3-(4-Allyloxyphenyl)-5,7-di-tert-butyl-benzofuran-2-on werden unter Stickstoffatmosphäre während ca. 4 Stunden bei 220°C gehalten. Anschliessend wird abgekühlt, mit 10 ml Essigsäure und 0,3 ml Methansulfonsäure versetzt und noch 7 Stunden am Rückfluss gekocht. Nach dem Verdünnen mit 100 ml Wasser wird das Produkt mit Dichlormethan extrahiert. Die organischen Phasen werden mit Wasser gewaschen, vereinigt, über Natriumsulfat getrocknet und am Vakuumrotationsverdampfer eingeengt. Chromatographie des Rückstandes an Kieselgel mit dem Laufmittelsystem Dichlormethan/Hexan = 1:2 und Kristallisation der reinen Fraktionen aus Ligroin liefert 1.07 g (28 %) 5,7-Di-tert-butyl-3-(2-methyl-dihydrobenzofuran-5-yl)benzofuran-2-on, Smp. 136-152°C als Diastereomeren-Gemisch (Verbindung (101), Tabelle 1).

Das für die Umsetzung benötigte 3-(4-Allyloxyphenyl)-5,7-di-tert-butyl-benzofuran-2-on lässt sich folgendermassen herstellen:

Ein Gemisch von 309 g (1,50 Mol) 2,4-Di-tert-butyl-phenol und 192 g (1,00 Mol) 4-Allyloxymandelsäure (hergestellt nach EP-B-182507, Beispiel 1, Seite 4) wird unter Stickstoffatmosphäre während 2 Stunden bei 140-150°C gerührt. Anschliessend wird unter leichtem Vakuum (50 mbar) noch während 1,5 Stunden bei 150°C nachgerührt. Das überschüssige 2,4-Di-tert-butyl-phenol wird am Hochvakuum abdestilliert. Kristallisation des Rückstandes aus Xylol und Ethanol liefert 230,1 g (61 %) 3-(4-Allyloxyphenyl)-5,7-di-tert-butylbenzofuran-2-on, Smp. 112-114°C.

Herstellung von 4-Allyoxy-mandelsäuren:

20,8 g (0,10 Mol) 4-Hydroxymandelsäure-Natriumsalz-Monohydrat und 6,6 g (0,10 Mol) Kaliumhydroxid werden mit 1,0 g (6,7 mMol) Natriumiodid in 75 ml Methanol gelöst. Danach wird 0,12 Mol Allylbromid (im Falle des Methallyls wird Methallylchlorid verwendet) zugegeben und unter Stickstoffatmosphäre während 16 Stunden unter Rückfluss gekocht. Das Reaktionsgemisch wird am Vakuumrotationsverdampfer eingeengt und der Rückstand mit konzentrierter Salzsäure angesäuert. Das Produkt wird mit Butylacetat dreimal extrahiert. Die organischen Phasen werden mit Wasser gewaschen, vereinigt, über Natriumsulfat getrocknet und am Vakuumrotationsverdampfer eingeengt. Kristallisation des Rückstandes aus Toluol/Benzin liefert die 4-Allyloxy-mandelsäuren, wie z.B. 4-Allyloxy-mandelsäure, amorphes Pulver (70 %) und 4-Methallyloxy-mandelsäure, Smp. 121-126° (65 %).

Herstellung substituierter 4-Hydroxy-mandelsäuren:

0,30 Mol Ausgangsphenol (beispielsweise o-Kresol, 2-tert-Butyl-phenol oder 2-Isopropyl-3-methyl-phenol) wird in 150 ml 2N Natriumhydroxid-Lösung unter Stickstoffatmosphäre gelöst. Nach Abkühlen auf +5°C werden 4,8 g (0,12 Mol) Natriumhydroxid und 13,3 ml (0,12 Mol) 50 %wässrige Glyoxylsäure zugegeben und das Reaktionsgemisch während 4 Stunden bei Raumtemperatur gerührt. Nach jeweils 4 Stunden werden zweimal weitere 0,12 Mol Natriumhydroxid und Glyoxylsäure zugegeben (total 0,36 Mol). Das Reaktionsgemisch wird anschliessend noch 12 Stunden gerührt, dann mit konzentrierter Salzsäure neutralisiert und mit zweimal 75 ml Petrolether gewaschen. Die wässrige Phase wird nun mit konzentrierter Salzsäure angesäuert und mit Ether mehrmals extrahiert. Die organischen Phasen werden vereinigt, über Magnesiumsulfat getrocknet und am Vakuumrotationsverdampfer eingeengt. Es werden so die folgenden Präparate erhalten: 4-Hydroxy-3-methyl-mandelsäure, Smp. 115-120°C, Ausbeute 55 %; 4-Hydroxy-3-tert-butylmandelsäure, Smp. 156-158°C, Ausbeute 26 %; und 3-Isopropyl-4-hydroxy-2-methylmandelsäure, Smp. 114-119°C, Ausbeute 20 %.

Beispiel 2: Herstellung von 3-(2,2-Dimethyl-dihydrobenzofuran-5-yl)-5,7-di-tert-butylbenzofuran-2-on (Verbindung (102), Tabelle 1).

Ein Gemisch von 20,6 g (0,10 Mol) 2,4-Di-tert-butyl-phenol und 22,6 g (0,10 Mol) 4-Methallyloxy-mandelsäure (Herstellung siehe Beispiel 1) wird unter vermindertem Druck (50 mbar) während 8 Stunden bei 155°C gehalten. Das Reaktionsgemisch wird anschliessend unter Stickstoffatmosphäre auf 220°C erwärmt und während ca. 100 Minuten bei dieser Temperatur gehalten. Nach dem Abkühlen auf etwa 120°C werden 100 ml Essigsäure und 2 ml Methansulfonsäure zugegeben und die Lösung noch während 3 Stunden unter Rückfluss gekocht. Das Reaktionsgemisch wird abgekühlt, mit 10 ml Ethanol versetzt und das Produkt filtriert. Umkristallisation aus Ethanol(100 ml)/Essigsäure (8 ml) liefert 8,0 g (20 %) 3-(2,2-Dimethyl-dihydrobenzofuran-5-yl)-5,7-di-tert-butyl-benzofuran-2-on, Smp. 164-166°C (Verbindung (102), Tabelle 1).

In Analogie zu Beispiel 2 werden aus den entsprechenden Phenolen und 4-Methallyloxymandelsäure die

Verbindungen (103) und (104) hergestellt. Bei der Herstellung von Verbindung (104) werden zwei Aequivalente 4-Methallyloxy-mandelsäure bezüglich eingesetztem Bishenol verwendet.

Beispiel 3: Herstellung von 3,3'-Bis-[3-(2,2-dimethyl-dihydrobenzofuran-5-yl)-5,7-di-tert-butyl-benzofuran-2-on] (Verbindung (105), Tabelle 1).

Zu einer Natriummethylat-Lösung, hergestellt durch Zugabe von 0,69 g (30,0 mMol) Natrium in 120 ml absoluten Methanol, wird unter Stickstoffatmosphäre 11,77 g (30 mMol) 3-(2,2-Dimethyl-dihydrobenzofuran-5-yl)-5,7-di-tert-butyl-benzofuran-2-on (Verbindung (102), Beispiel 2) gegeben. Anschliessend wird bei Raumtemperatur während ca. 10 Minuten eine Lösung von 3,8 g (15,0 mMol) Jod in 60 ml Diethylether zugetropft. Das Reaktionsgemisch wird noch 30 Minuten nachgerührt, danach mit 500 ml Wasser verdünnt und dreimal mit je 100 ml Diethylether extrahiert. Die organischen Phasen werden abgetrennt, mit Wasser gewaschen, vereinigt, über Magnesiumsulfat getrocknet und am Vakuumrotationsverdampfer eingeengt. Zweimalige Kristallisation des Rückstandes aus Methanol liefert 3,1 g (26 %) des 3,3'-Bis-[3-(2,2-dimethyl-dihydrobenzofuran-5-yl)-5,7-di-tert-butylbenzofuran-2-on] Smp. 202-210°C (Verbindung (105), Tabelle 1).

Tabelle 1:

| Nr. | Verbindung | Smp. (°C) | C (%), H (%) (berechnet/gefunden) | Ausbeute (%) |
|---|---|---|---|---|
| 101 | 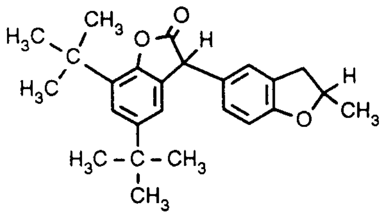 | 136-152 | 79,33 7,99<br>79,34 7,99<br>Diastereomeren-Gemisch | 28 |
| 102 | 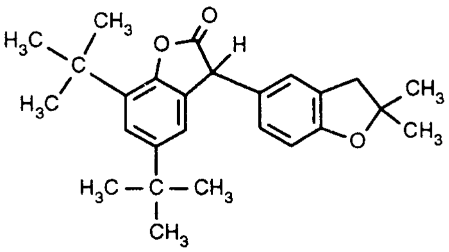 | 164-166 | 79,56 8,22<br>79,33 8,35 | 20 |
| 103 | 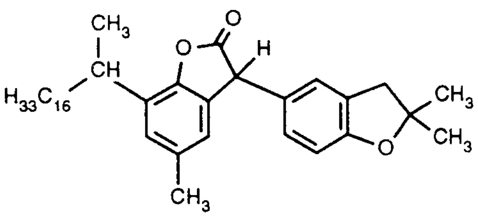 | 69-86 | 81,27 9,95<br>81,27 9,93<br>Diastereomeren-Gemisch | 50 |
| 104 | 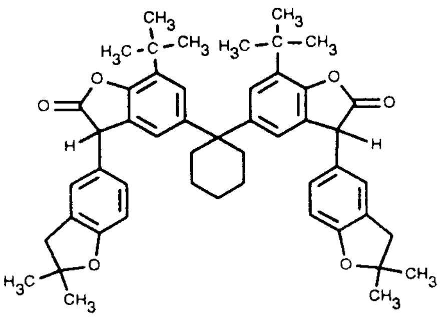 | Harz | Charakterisiert durch MS:<br>für $C_{50}H_{56}O_6$<br>berechnet: $M^+$ = 752,99<br>gefunden: $M^+$ = 753,0<br>Diastereomeren-Gemisch | 75 |

Tabelle 1: (Fortsetzung)

| Nr. | Verbindung | Smp. (°C) | C (%), H (%) (berechnet/gefunden) | Ausbeute (%) |
|---|---|---|---|---|
| 105 | 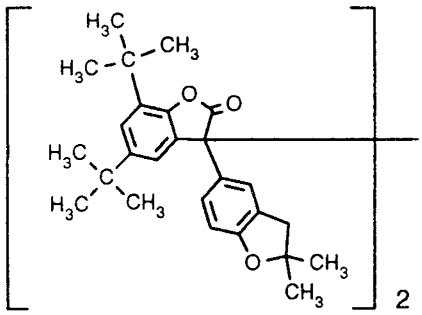 | 202-210 | 79,97 7,74<br>79,53 7,96 | 26 |

Beispiel 4: Stabilisierung von Polypropylen bei Mehrfachextrusion.

1,3 kg Polypropylenpulver (Profax 6501), das mit 0,025 % Irganox® 1076 (3-[3,5-Di-tert-butyl-4-hydroxyphenyl]propionsäure-n-octadecylester) vorstabilisiert wurde (mit einem bei 230°C und mit 2,16 kg gemessenen Schmelzindex von 3,2), wird gemischt mit 0,05 % Irganox® 1010 (Pentaerythrit-tetrakis-[3-(3,5-di-tert-butyl-4-hydroxyphenyl)-propionat), 0,05 % Calciumstearat, 0,03 % DHT 4A® (Kyowa Chemical Industry Co., Ltd., $[Mg_{4.5}Al_2(OH)_{13}CO_3 \cdot 3,5\ H_2O]$) und 0,015 % Verbindung aus Tabelle 1. Diese Mischung wird in einem Extruder mit einem Zylinderdurchmesser von 20 mm und einer Länge von 400 mm mit 100 Umdrehungen pro Minute extrudiert, wobei die 3 Heizzonen auf die folgenden Temperaturen eingestellt werden: 260, 270, 280°C. Das Extrudat wird zur Kühlung durch ein Wasserbad gezogen und anschliessend granuliert. Dieses Granulat wird wiederholt extrudiert. Nach 3 Extrusionen wird der Schmelzindex gemessen (bei 230°C mit 2,16 kg). Grosse Zunahme des Schmelzindex bedeutet starken Kettenabbau, also schlechte Stabilisierung. Die Resultate sind in Tabelle 2 zusammengefasst.

Tabelle 2

| Verbindung aus Tabelle 1 | Schmelzindex nach 3 Extrusionen |
|---|---|
| - | 20,0 |
| 101 | 5,3 |
| 102 | 5,9 |
| 103 | 5,6 |

Beispiel 5: Stabilisierung von Polyethylen während der Verarbeitung.

100 Teile Polyethylenpulver (Lupolen® 5260 Z) werden mit 0,05 Teilen Pentaerythrittetrakis-[3-(3,5-di-tert-butyl-4-hydroxyphenyl)-propionat], 0,05 Teilen Tris-(2,4-di-tert-butyl-phenyl)-phosphit und 0,05 Teilen Verbindung aus Tabelle 1 gemischt und in einem Brabender Plastographen bei 220°C und 50 Umdrehungen pro Minute geknetet. Während dieser Zeit wird der Knetwiderstand als Drehmoment kontinuierlich registriert. Im Verlauf der Knetzeit beginnt das Polymere nach längerer Konstanz zu vernetzen, was anhand der raschen Zunahme des Drehmoments festgestellt werden kann. In der Tabelle 3 ist die Zeit bis zur merklichen Zunahme des Drehmoments als Mass der Stabilisatorwirkung angegeben. Je länger diese Zeit ist, desto besser ist die Stabilisatorwirkung.

Tabelle 3

| Verbindung aus Tabelle 1 | Zeit bis zum Anstieg von Drehmoment (Min) |
|---|---|
| - | 9,0 |
| 101 | 34,5 |
| 102 | 28,5 |
| 103 | 32,5 |

**Patentansprüche**

**1.** Verbindungen der Formel (1)

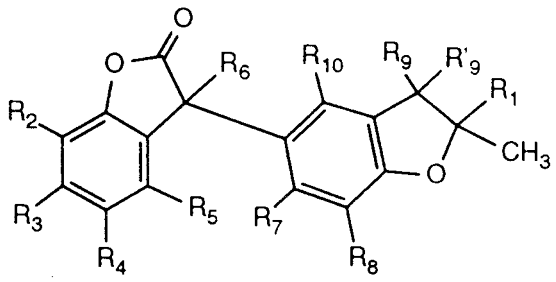
(1)

worin

$R_1$ Wasserstoff, $C_1$-$C_4$-Alkyl, unsubstituiertes oder durch $C_1$-$C_4$-Alkyl substituiertes Phenyl bedeutet,
$R_2$, $R_3$, $R_4$ und $R_5$ unabhängig voneinander Wasserstoff, Chlor, $C_1$-$C_{25}$-Alkyl, $C_7$-$C_9$-Phenylalkyl, unsubstituiertes oder durch $C_1$-$C_4$-Alkyl substituiertes Phenyl, unsubstituiertes oder durch $C_1$-$C_4$-Alkyl substituiertes $C_5$-$C_8$-Cycloalkyl; $C_1$-$C_{18}$-Alkoxy, $C_1$-$C_{18}$-Alkylthio, Hydroxy, $C_1$-$C_4$-Alkylamino, Di-($C_1$-$C_4$-alkyl)amino, $C_1$-$C_{25}$-Alkanoyloxy, $C_1$-$C_{25}$-Alkanoylamino, $C_3$-$C_{25}$-Alkenoyloxy, durch Sauerstoff, Schwefel oder >N-$R_{16}$ unterbrochenes $C_3$-$C_{25}$-Alkanoyloxy; $C_6$-$C_9$-Cycloalkylcarbonyloxy, Benzoyloxy oder durch $C_1$-$C_{12}$-Alkyl substituiertes Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Phenylring bilden, $R_4$ zusätzlich -$(CH_2)_n$-$COR_{11}$ darstellt, oder wenn $R_3$, $R_5$, $R_6$, $R_7$ und $R_{10}$ Wasserstoff sind, $R_4$ zusätzlich einen Rest der Formel (2)

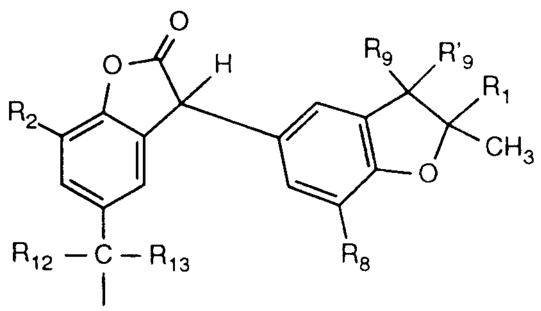
(2)

bedeutet,
$R_6$ Wasserstoff oder einen Rest der Formel (3)

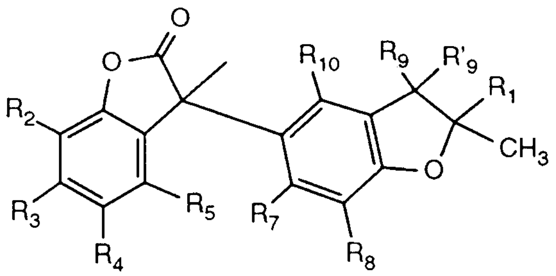 (3)

darstellt, wobei $R_4$ nicht einen Rest der Formel (2) bedeutet,
$R_7$ und $R_8$ unabhängig voneinander Wasserstoff oder $C_1$-$C_4$-Alkyl darstellen,
$R_9$ und $R'_9$ Wasserstoff, $C_1$-$C_4$-Alkyl oder Phenyl bedeuten, mit der Bedingung, dass mindestens einer der Reste $R_9$ und $R'_9$ Wasserstoff ist,
$R_{10}$ Wasserstoff oder $C_1$-$C_4$-Alkyl darstellt,
$R_{11}$ Hydroxy, $[-O^{\ominus}\frac{1}{r}\ M^{r+}]$, $C_1$-$C_{18}$-Alkoxy oder

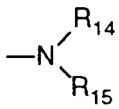

bedeutet,
$R_{12}$ und $R_{13}$ unabhängig voneinander Wasserstoff, $CF_3$, $C_1$-$C_{12}$-Alkyl oder Phenyl darstellen, oder $R_{12}$ und $R_{13}$ zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden;
$R_{14}$ und $R_{15}$ unabhängig voneinander Wasserstoff oder $C_1$-$C_{18}$-Alkyl darstellen,
$R_{16}$ Wasserstoff oder $C_1$-$C_8$-Alkyl bedeutet,
M ein r-wertiges Metallkation ist,
n 0, 1 oder 2 und
r 1,2 oder 3 bedeutet.

**2.** Verbindungen gemäss Anspruch 1, worin

$R_1$ Wasserstoff, $C_1$-$C_4$-Alkyl oder Phenyl bedeutet,
$R_2$, $R_3$, $R_4$ und $R_5$ unabhängig voneinander Wasserstoff, Chlor, $C_1$-$C_{18}$-Alkyl, Benzyl, Phenyl, $C_5$-$C_8$-Cycloalkyl, $C_1$-$C_{18}$-Alkoxy, $C_1$-$C_{18}$-Alkylthio, $C_1$-$C_{18}$-Alkanoyloxy, $C_1$-$C_{18}$-Alkanoylamino, $C_3$-$C_{18}$-Alkenoyloxy oder Benzoyloxy darstellen, oder ferner die Reste $R_2$ und $R_3$ oder die Reste $R_4$ und $R_5$ zusammen mit den Kohlenstoffatomen, an die sie gebunden sind, einen Phenylring bilden, oder wenn $R_3$, $R_5$, $R_6$, $R_7$ und $R_{10}$ Wasserstoff sind, $R_4$ zusätzlich einen Rest der Formel (2) bedeutet,
$R_9$ und $R'_9$ Wasserstoff oder $C_1$-$C_4$-Alkyl darstellen, mit der Bedingung, dass mindestens einer der Reste $R_9$ und $R'_9$ Wasserstoff ist, und
$R_{12}$ und $R_{13}$ entweder Methylgruppen sind, oder zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden.

**3.** Verbindungen gemäss Anspruch 1, worin mindestens zwei der Reste $R_2$, $R_3$, $R_4$ und $R_5$ Wasserstoff sind.

**4.** Verbindungen gemäss Anspruch 1, worin $R_3$ und $R_5$ Wasserstoff sind.

**5.** Verbindungen gemäss Anspruch 1, worin

$R_1$ Wasserstoff oder $C_1$-$C_4$-Alkyl bedeutet,
$R_2$ und $R_4$ unabhängig voneinander Wasserstoff, $C_1$-$C_{18}$-Alkyl, Benzyl, Phenyl, $C_5$-$C_8$-Cycloalkyl oder $C_1$-$C_{18}$-Alkoxy darstellen, oder wenn $R_3$, $R_5$, $R_6$, $R_7$ und $R_{10}$ Wasserstoff sind, $R_4$ zusätzlich einen Rest der Formel (2)

bedeutet,

$R_3$ und $R_5$ unabhängig voneinander Wasserstoff oder $C_1$-$C_4$-Alkyl darstellen,
$R_6$ Wasserstoff oder einen Rest der Formel (3) darstellt, wobei $R_4$ nicht einen Rest der Formel (2) bedeutet,
$R_7$, $R_8$, $R_9$, $R'_9$ und $R_{10}$ Wasserstoff sind, und
$R_{12}$ und $R_{13}$ entweder Methylgruppen sind, oder zusammen mit dem C-Atom, an das sie gebunden sind, einen $C_5$-$C_8$-Cycloalkylidenring bilden.

**6.** Verbindungen gemäss Anspruch 1, worin

$R_1$ Wasserstoff oder Methyl ist,
$R_2$ Wasserstoff oder $C_1$-$C_{18}$-Alkyl bedeutet,
$R_3$, $R_5$, $R_7$, $R_8$, $R_9$, $R'_9$ und $R_{10}$ Wasserstoff sind,
$R_4$ Wasserstoff oder $C_1$-$C_4$-Alkyl darstellt, oder wenn $R_6$ Wasserstoff ist, $R_4$ zusätzlich einen Rest der Formel (2) bedeutet,
$R_6$ Wasserstoff oder einen Rest der Formel (3) darstellt, wobei $R_4$ nicht einen Rest der Formel (2) bedeutet, und
$R_{12}$ und $R_{13}$ entweder Methylgruppen sind, oder zusammen mit dem C-Atom, an das sie gebunden sind, einen Cyclohexylidenring bilden.

**7.** Zusammensetzung enthaltend

a) ein dem oxidativen, thermischen oder lichtinduzierten Abbau unterworfenes organisches Material und
b) mindestens eine Verbindung der Formel (1) gemäss Anspruch 1.

**8.** Zusammensetzung gemäss Anspruch 7, worin die Komponente a) ein synthetisches Polymer ist.

**9.** Zusammensetzung gemäss Anspruch 7, worin die Komponente b) in einer Menge von 0,0005 bis 5 % bezogen auf das Gewicht der Komponente a) vorliegt.

**10.** Zusammensetzung gemäss Anspruch 7, enthaltend zusätzlich ein organisches Phosphit oder Phosphonit.

**11.** Verwendung der Verbindungen der in Anspruch 1 definierten Formel (1) als Stabilisatoren für organische Materialien gegen oxidativen, thermischen oder lichtinduzierten Abbau.

**12.** Verfahren zum Stabilisieren eines organischen Materials gegen oxidativen, thermischen oder lichtinduzierten Abbau, dadurch gekennzeichnet, dass man diesem mindestens eine Verbindung der in Anspruch 1 definierten Formel (1) einverleibt oder auf dieses aufbringt.

**Claims**

**1.** A compound of formula (1)

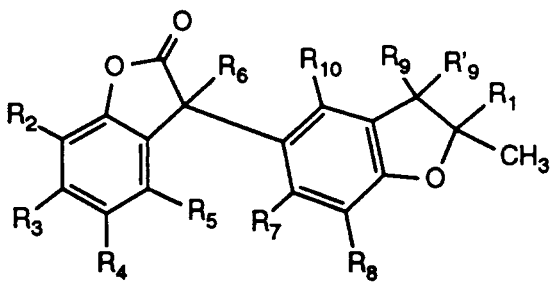

(1)

wherein

$R_1$ is hydrogen, $C_1$-$C_4$alkyl, unsubstituted or $C_1$-$C_4$alkyl-substituted phenyl,
$R_2$, $R_3$, $R_4$ and $R_5$ are each independently of one another hydrogen, chloro, $C_1$-$C_{25}$alkyl, $C_7$-$C_9$phenylalkyl,

unsubstituted or $C_1$-$C_4$alkyl-substituted phenyl, unsubstituted or $C_1$-$C_4$alkyl-substituted $C_5$-$C_8$cycloalkyl; $C_1$-$C_{18}$alkoxy, $C_1$-$C_{18}$alkylthio, hydroxy, $C_1$-$C_4$alkylamino, di($C_1$-$C_4$alkyl)amino, $C_1$-$C_{25}$alkanoyloxy, $C_1$-$C_{25}$alkanoylamino, $C_3$-$C_{25}$alkenoyloxy, $C_3$-$C_{25}$alkanoyloxy which is interrupted by oxygen, sulfur or $>N-R_{16}$ $C_6$-$C_9$cycloalkylcarbonyloxy, benzoyloxy or $C_1$-$C_{12}$alkyl-substituted benzoyloxy, or $R_2$ and $R_3$ or $R_3$ and $R_4$ or $R_4$ and $R_5$, together with the carbon atoms to which they are attached, form a phenyl ring, $R_4$ is additionally -$(CH_2)_n$-$COR_{11}$, or, if $R_3$, $R_5$, $R_6$, $R_7$ and $R_{10}$ are hydrogen, $R_4$ is additionally a radical of formula (2)

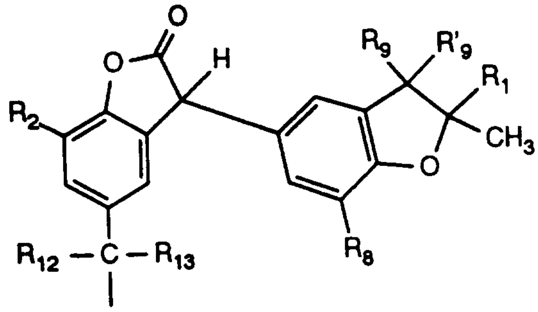

(2)

$R_6$ is hydrogen or a radical of formula (3)

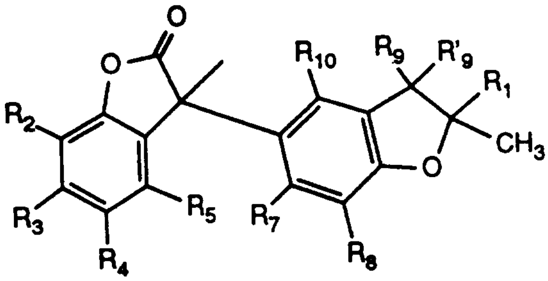

(3)

wherein $R_4$ is not a radical of formula (2),
$R_7$ and $R_8$ are each independently of other hydrogen or $C_1$-$C_4$alkyl,
$R_9$ and $R'_9$ are hydrogen, $C_1$-$C_4$alkyl or phenyl, with the proviso that at least one of $R_9$ and $R'_9$ is hydrogen,
$R_{10}$ is hydrogen or $C_1$-$C_4$alkyl,
$R_{11}$ is hydroxy, $[-O^{\ominus}\frac{1}{r}M^{r+}]$ $C_1$-$C_{18}$alkoxy or,

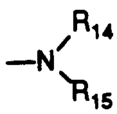

$R_{12}$ and $R_{13}$ are each independently of one other hydrogen, $CF_3$, $C_1$-$C_{12}$alkyl or phenyl, or $R_{12}$ and $R_{13}$, together with the carbon atom, to which they are attached, form a $C_5$-$C_8$cycloalkylidene ring which is unsubstituted or substituted by 1 to 3 $C_1$-$C_4$alkyl groups;
$R_{14}$ and $R_{15}$ are each independently of one other hydrogen or $C_1$-$C_{18}$alkyl,
$R_{16}$ is hydrogen or $C_1$-$C_8$alkyl,
M is a metal cation of valency r,
n is 0, 1 or 2, and
r is 1, 2 or 3.

**2.** A compound according to claim 1, wherein

$R_1$ is hydrogen, $C_1$-$C_4$alkyl or phenyl,

$R_2$, $R_3$, $R_4$ and $R_5$ are each independently of one another hydrogen, chloro, $C_1$-$C_{18}$alkyl, benzyl, phenyl, $C_5$-$C_8$cycloalkyl, $C_1$-$C_{18}$alkoxy, $C_1$-$C_{18}$alkylthio, $C_1$-$C_{18}$alkanoyloxy, $C_1$-$C_{18}$alkanoylamino, $C_3C_{18}$alkenoyloxy or benzoyloxy, or $R_2$ and $R_3$ or $R_4$ and $R_5$, together with the carbon atoms to which they are attached, form a phenyl ring, or, if $R_3$, $R_5$, $R_6$, $R_7$ and $R_{10}$ are hydrogen, $R_4$ is additionally a radical of formula (2),
$R_9$ and $R'_9$ are hydrogen or $C_1$-$C_4$alkyl, with the proviso that at least one of $R_9$ and $R'_9$ is hydrogen, and
$R_{12}$ and $R_{13}$ are either methyl groups or, together with the carbon atom to which they are attached, form a $C_5$-$C_8$cycloalkylidene ring which is unsubstituted or substituted by 1 to 3 $C_1$-$C_4$alkyl groups.

**3.** A compound according to claim 1, wherein at least two of $R_2$, $R_3$, $R_4$ and $R_5$ are hydrogen.

**4.** A compound according to claim 1, wherein $R_3$ and $R_5$ are hydrogen.

**5.** A compound according to claim 1, wherein

$R_1$ is hydrogen or $C_1$-$C_4$alkyl,
$R_2$ and $R_4$ are each independently of one other hydrogen, $C_1$-$C_{18}$alkyl, benzyl, phenyl, $C_5$-$C_8$cycloalkyl or $C_1$-$C_{18}$alkoxy, or, if $R_3$, $R_5$, $R_6$, $R_7$ and $R_{10}$ are hydrogen, $R_4$ is additionally a radical of formula (2),
$R_3$ and $R_5$ are each independently of the other hydrogen or $C_1$-$C_4$alkyl,
$R_6$ is hydrogen or a radical of formula (3), where $R_4$ is not a radical of formula (2),
$R_7$, $R_8$, $R_9$, $R'_9$ and $R_{10}$ are hydrogen, and
$R_{12}$ and $R_{13}$ are either methyl groups or, together with the carbon atom to which they are attached, form a $C_5$-$C_8$cycloalkylidene ring.

**6.** A compound according to claim 1, wherein

$R_1$ is hydrogen or methyl,
$R_2$ is hydrogen or $C_1$-$C_{18}$alkyl,
$R_3$, $R_5$, $R_7$, $R_8$, $R_9$, $R'_9$ and $R_{10}$ are hydrogen,
$R_4$ is hydrogen or $C_1$-$C_4$alkyl, or, if $R_6$ is hydrogen, $R_4$ is additionally a radical of formula (2),
$R_6$ is hydrogen or a radical of formula (3), wherein $R_4$ is not a radical of formula (2), and
$R_{12}$ and $R_{13}$ are either methyl groups or, together with the carbon atom to which they are attached, form a cyclohexylidene ring.

**7.** A composition comprising

a) an organic material which is subject to oxidative, thermal or light-induced degradation, and
b) at least one compound of formula (1) according to claim 1.

**8.** A composition according to claim 7, wherein component a) is a synthetic polymer.

**9.** A composition according to claim 7, which contains 0.0005 to 5% of component b), based on the weight of component a).

**10.** A composition according to claim 7, which additionally comprises an organic phosphite or phosphonite.

**11.** The use of a compound of formula (1) as defined in claim 1 for stabilizing organic materials against oxidative, thermal or light-induced degradation.

**12.** A process for stabilizing an organic material against oxidative, thermal or light-induced degradation, which comprises incorporating therein or applying thereto at least one compound of formula (1) as defined in claim 1.

**Revendications**

**1.** Composés de formule (1)

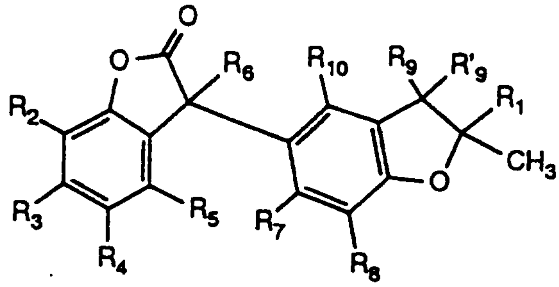

(1)

où

$R_1$ représente un atome d'hydrogène, des groupes alkyle en $C_1$-$C_4$, phényle non substitué ou substitué par des groupes alkyle en $C_1$-$C_4$,

$R_2$, $R_3$, $R_4$ et $R_5$ représentent, indépendamment les uns des autres, des atomes d'hydrogène, des atomes de chlore, des groupes alkyle en $C_1$-$C_{25}$, phénylalkyle en $C_7$-$C_9$, phényle non substitué ou substitué par des groupes alkyle en $C_1$-$C_4$, cycloalkyle en $C_5$-$C_8$ non substitué ou substitué par des groupes alkyle en $C_1$-$C_4$ ; alkoxy en $C_1$-$C_{18}$, (alkyl en $C_1$-$C_{18}$)thio, hydroxy, (alkyl en $C_1$-$C_4$)amino, di-(alkyl en $C_1$-$C_4$)amino, (alcanoyl en $C_1$-$C_{25}$)oxy, (alcanoyl en $C_1$-$C_{25}$)amino, (alcénoyl en $C_3$-$C_{25}$)-oxy, (alcanoyl en $C_3$-$C_{25}$)oxy interrompu par des atomes d'oxygène, de soufre ou un groupe >N-$R_{16}$; (cycloalkyl en $C_6$-$C_9$ )carbonyloxy, benzoyloxy ou benzoyloxy substitué par un groupe alkyle en $C_1$-$C_{12}$, ou de plus les restes $R_2$ et $R_3$ ou les restes $R_3$ et $R_4$ ou les restes $R_4$ et $R_5$ forment, conjointement avec les atomes de carbone auxquels ils sont liés, un cycle phényle, $R_4$ représente de plus un groupe $-(CH_2)_n-COR_{11}$, ou lorsque $R_3$, $R_5$, $R_6$, $R_7$ et $R_{10}$ représentent des atomes d'hydrogène, $R_4$ représente de plus un reste de formule (2)

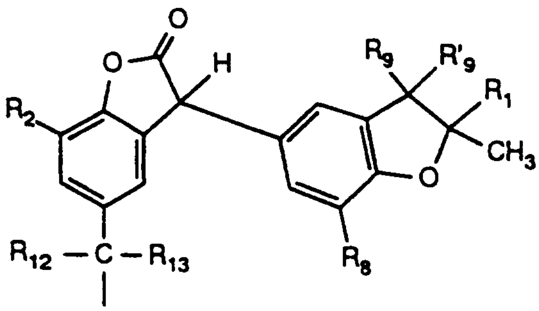

(2)

$R_6$ représente un atome d'hydrogène ou un reste de formule (3)

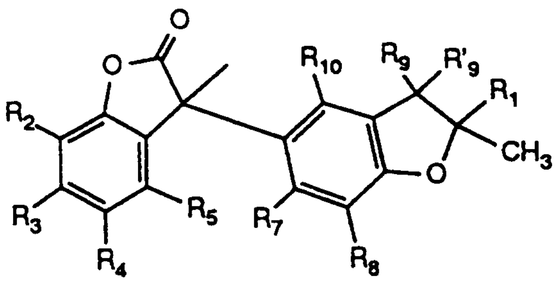

(3)

$R_4$ ne représentant pas un reste de formule (2),

$R_7$ et $R_8$ représentent, indépendamment l'un de l'autre, des atomes d'hydrogène ou des groupes alkyle en $C_1$-$C_4$,

$R_9$ et $R'_9$ représentent des atomes d'hydrogène, des groupes alkyle en $C_1$-$C_4$ ou phényle, à la condition qu'au moins l'un des restes $R_9$ et $R'_9$ représente un atome d'hydrogène,
$R_{10}$ représente un atome d'hydrogène ou un groupe alkyle en $C_1$-$C_4$,
$R_{11}$ représente un groupe hydroxy, $[-O^{\ominus}\frac{1}{r}M^{r+}]$, alkoxy en $C_1$-$C_{18}$ ou

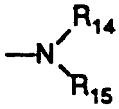

,

$R_{12}$ et $R_{13}$ représentent, indépendamment l'un de l'autre, des atomes d'hydrogène, des groupes $CF_3$, alkyle en $C_1$-$C_{12}$ ou phényle, ou $R_{12}$ et $R_{13}$ forment, conjointement avec l'atome de carbone auquel ils sont liés, un cycle cycloalkylidène non substitué ou substitué par 1 à 3 groupes alkyle en $C_1$-$C_4$ ;
$R_{14}$ et $R_{15}$ représentent, indépendamment l'un de l'autre, des atomes d'hydrogène ou des groupes alkyle en $C_1$-$C_{18}$ ;
$R_{16}$ représente un atome d'hydrogène ou un groupe alkyle en $C_1$-$C_8$,
M représente un cation de métal à fonctionnalité r,
n vaut 0, 1 ou 2,
r vaut 1, 2 ou 3.

**2.** Composés selon la revendication 1, où

$R_1$ représente un atome d'hydrogène, des groupes alkyle en $C_1$-$C_4$ ou phényle,
$R_2$, $R_3$, $R_4$ et $R_5$ représentent, indépendamment les uns des autres, des atomes d'hydrogène, des atomes de chlore, des groupes alkyle en $C_1$-$C_{18}$, benzyle, phényle, cycloalkyle en $C_3$-$C_8$, alkoxy en $C_1$-$C_{18}$, (alkyl en $C_1$-$C_{18}$)thio, (alcanoyl en $C_1$-$C_{18}$)oxy, (alcanoyl en $c_1$-$c_{18}$)amino, (alcénoyl en $C_3$-$c_{18}$)-oxy ou benzoyloxy, ou de plus les restes $R_2$ et $R_3$ ou les restes $R_4$ et $R_5$ forment, conjointement avec les atomes de carbone auxquels ils sont liés, un cycle phényle, ou lorsque $R_3$, $R_5$, $R_6$, $R_7$ et $R_{10}$ représentent des atomes d'hydrogène, $R_4$ représente de plus un reste de formule (2),
$R_9$ et $R_9$ représentent des atomes d'hydrogène ou des groupes alkyle en $C_1$-$C_4$, à la condition qu'au moins l'un des restes $R_9$ et $R_9$ représente un atome d'hydrogène,
$R_{12}$ et $R_{13}$ représentent soit des groupes méthyle, soit forment, conjointement avec l'atome de carbone auquel ils sont liés, un groupe cycloalkyle en $C_3$-$C_8$ non substitué ou substitué par 1 à 3 groupes alkyle en $C_1$-$C_4$.

**3.** Composés selon la revendication 1, où au moins deux des restes $R_2$, $R_3$, $R_4$ et $R_5$ représentent des atomes d'hydrogène.

**4.** Composés selon la revendication 1, où $R_3$ et $R_5$ représentent des atomes d'hydrogène.

**5.** Composés selon la revendication 1, où

$R_1$ représente un atome d'hydrogène ou un groupe alkyle en $C_1$-$C_4$,
$R_2$ et $R_4$ représentent, indépendamment l'un de l'autre, des atomes d'hydrogène, des groupes alkyle en $C_1$-$C_{18}$, benzyle, phényle, cycloalkyle en $C_5$-$C_8$ ou alkoxy en $C_1$-$C_{18}$, ou lorsque $R_3$, $R_5$, $R_6$, $R_7$ et $R_{10}$ représentent des atomes d'hydrogène, $R_4$ représente de plus un reste de formule (2),
$R_3$ et $R_5$ représentent, indépendamment l'un de l'autre, des atomes d'hydrogène ou un groupe alkyle en $C_1$-$C_4$,
$R_6$ représente un atome d'hydrogène ou un reste de formule (3), $R_4$ ne signifiant pas un reste de formule (2),
$R_7$, $R_8$, $R_9$, $R_9$ et $R_{10}$ représentent des atomes d'hydrogène, et
$R_{12}$ et $R_{13}$ représentent soit un groupe méthyle, soit forment, conjointement avec l'atome de carbone auquel ils sont liés, un cycle cycloalkylidène en $C_5$-$C_8$.

**6.** Composés selon la revendication 1, où

$R_1$ représente un atome d' hydrogène ou un groupe méthyle,
$R_2$ représente un atome d'hydrogène ou un groupe alkyle en $C_1$-$C_{18}$,
$R_3$, $R_5$, $R_7$, $R_8$, $R_9$, $R_9$ et $R_{10}$ représentent des atomes d'hydrogène,

$R_4$ représente un atome d'hydrogène ou un groupe alkyle en $C_1$-$C_4$, ou lorsque $R_6$ représente un atome d'hydrogène, $R_4$ représente de plus un reste de formule (2),

$R_6$ représente un atome d'hydrogène ou un reste de formule (3), $R_4$ ne signifiant pas un reste de formule (2), et $R_{12}$ et $R_{13}$ représentent soit des groupes méthyle, soit forment, conjointement avec l'atome de carbone auquel ils sont liés, un cycle cyclohexylidène.

**7.** Composition contenant

a) une matière organique soumise la dégradation oxydative, thermique ou induite par la lumière,
b) au moins un composé de formule (1), selon la revendication 1.

**8.** Composition selon la revendication 7, où le composant a) est un polymère synthétique.

**9.** Composition selon la revendication 7, où le composant b) est présent en une quantité de 0,0005 à 5% par rapport au poids du composant a).

**10.** Composition selon la revendication 7, contenant de plus un phosphite ou phosphonite organique.

**11.** Utilisation des composés de formule (1) définie à la revendication 1, comme stabilisants de matières organiques contre la dégradation oxydative, thermique ou induite par la lumière.

**12.** Procédé de stabilisation d'une matière organise contre la dégradation oxydative, thermique ou induite par la lumière, caractérisé en ce qu'on incorpore à ou on applique sur celle-ci au moins un composé de formule (1) définie à la revendication 1.